# EUROPEAN PATENT SPECIFICATION

(11) **EP 4 014 731 B1**
(45) Date of publication and mention of the grant of the patent: **05.08.2026**
(21) Application number: 20214873.0
(22) Date of filing: 17.12.2020
(51) Int. Cl.: B64D 1/18, B64U 10/14, F24F 6/00, F24F 8/00, F24F 8/10, A01M 1/20, A61L 9/20, A01M 5/00, A61L 9/12

(54) **DRONE ADAPTED FOR MODIFYING AIR PROPERTIES OF AN ENVIRONMENT AND CORRESPONDING METHOD**
ZUR VERÄNDERUNG VON LUFTEIGENSCHAFTEN EINER UMGEBUNG ANGEPASSTE DROHNE UND ENTSPRECHENDES VERFAHREN
DRONE ADAPTÉ POUR MODIFIER LES PROPRIÉTÉS DE L'AIR D'UN ENVIRONNEMENT ET PROCÉDÉ CORRESPONDANT

(43) Date of publication of application: 22.06.2022
(73) Proprietor: Henkel AG & Co. KGaA, 40589 Düsseldorf (DE)
(72) Inventor: Schaefer, Sascha, 40822 Mettmann (DE)

(56) References cited:
- WO-A1-2018/182944
- ES-U- 1 249 099
- KR-A- 20200 110 049
- US-A1- 2017 231 213
- US-A1- 2018 074 521
- US-A1- 2018 155 026

## Description

### TECHNICAL FIELD

The invention relates to the field of air refreshing systems and/or insect repellents that are mounted on drones or any other device capable of flying through air to modify air quality by dispensing a fragrance, filtering, sanitizing the air and/or dispensing a pest repellent. A wide range of air modification features can be included in the flying device such as air humidifiers, air filtering systems, pest repellents, air sanitizing, fragrance dispensers, air conditioning for example.

### TECHNOLOGICAL BACKGROUND

A person's well-being partly depends on the conditions of the environment in which the person lives. Temperature, humidity, smells, pollution, allergens or the presence of pest such as mosquitos, bugs, mites for example all affect a person's sense of well-being. Sometimes an environment can even become unhealthy and requires some action to modify air properties and remove pest, pollutants or allergens. Some pest also spreads disease and regulation of pest is an important issue for well-being and health.

Typically, changing air properties or removing pest from an environment is done using static devices that dispense some pest repellent or fragrance into the air from a device plugged into a socket.

Air conditioning systems, air humidifiers or air filtering devices are sometimes used in the form of bulky devices that are also stationary and remain plugged into a socket in a fixed position. All solutions provided by prior art rely on static systems that require a user to select a location from which air properties will be modified. This approach is not practical as it requires an electrical plug and does not offer enough flexibility to adapt to changing environments or to quickly relocate the device.

An autonomous device, capable of moving through the air without the need for contact with the surface for use as a device for purification, disinfection of micro-organisms and particles in the domestic or industrial environment and as a control element for air quality is described in ES 1 249 099 U. This flying drone is equipped with a sensor and detector elements necessary to quantify the purity and clean the air.

Some specific applications have been imagined in which flying drones are used as platforms for dispensing pest repellents based on a sensing of chemicals emitted by the pest themselves. Documents US 2017/0231213 A1 and US 2018/0074521 A1 describe one such example. Although this solution offers a better flexibility in dispensing pest repellents, this solution is not adapted to finely control the dispensing of the pest repellent in the environment and still relies on traditional dispensing techniques, the only difference being the mounting of the dispensing device on a mobile object.

For the above reasons, a device adapted for modifying air properties of an environment is sought.

### SUMMARY OF THE INVENTION

To address the above need, the invention provides a drone adapted for modifying air properties of an environment, the drone comprising:
- a body;
- at least one air ventilation mechanism connected to the body;
- an air circulation pathway arranged in the body, the air circulation pathway being configured to suck in air and to expel air upon activation of the at least one air ventilation mechanism;
- steering means configured to maneuver the drone in air;
- at least one air treatment portion adapted for modifying air properties, the at least one air treatment portion being arranged in the body along the air circulation pathway;
- a processor, the processor being configured to control the steering means and to control the at least one air ventilation mechanism;

wherein the processor is adapted to receive an instruction to modify air properties and
wherein the processor controls the steering means and controls the at least one air ventilation mechanism based on the instruction, so that the drone navigates through air in the environment and the at least one air ventilation mechanism creates a circulation of air through the at least one air treatment portion
wherein the at least one air ventilation mechanism comprises several air flow intensity settings for controlling flux of air channeled along the air circulation pathway and through the air treatment portion.

The drone of the invention not only offers flexibility of movement so that air properties can be changed locally without requiring direct intervention of a user, but also allows the air purification or dispensing of a product to occur more efficiently using the ventilation mechanism that creates a flow of air through an air treatment portion arranged along an air circulation pathway in the body of the drone. The presence of one or more air ventilation mechanisms also offers the possibility of a finer control of the flow of air through the air treatment portion. In this way, the speed at which the air quality is modified and/or the concentration of the product that is dispensed into the air can be controlled with more precision. Noise generated by the drone and battery power consumption can thus also be monitored and regulated. The invention offers a high degree of flexibility in the treatment of air properties and also offers some modularity by allowing the presence of more than one air treatment portion. Different actions can be performed on air to change its properties, either dispense a fragrance, filter the air, dispense a pest repellent, sanitize air using for example ultra violet (UV) radiation, heat or cool the air, humidify the air, dehumidify the air, absorb smells.

The processor of the drone of the invention determines a flight path that is compliant with the instruction it receives. This instruction can be input by a user (and for example determine a target to be reached: maintain pollutant levels below a certain threshold, eliminate mosquito disturbance, absorb smells or smoke generated from cooking appliances, or maintain air humidity below a certain level). The instruction can also be enhanced with local information about the weather, the presence of wet clothes, the usage of cooking appliances or detection of pest, allergens, pollutants, viruses or aerosols. The drone may operate according to an automatic patrolling mode or apply instructions it receives with few changes, the changes being for example limited to proximity warnings of the drone and emergency situations (such as hazardous detected air quality).

According to an embodiment the at least one air treatment portion may comprise a fragrance dispensing device.

According to an embodiment the at least one air treatment portion may comprise a pest repellent.

According to an embodiment the processor may further control dispensing of an air modifying composition in the at least one air treatment portion.

In particular the processor may be configured to determine and adapt a dosing of a fragrance, a pest repellent or humidifying composition based on input it receives either from a user or from a sensor. In case of input received from a sensor, the dosing strategy can be computed by applying dosing algorithms pre-stored in a memory of the drone to which the processor can have access.

According to the invention at least one air ventilation mechanism comprises several air flow intensity settings for controlling flux of air channeled along the air circulation pathway and through the air treatment portion.

Air flow intensity settings allow a user to control the speed at which air properties are changed and also offers some latitude in the dosage of air modifying agents that are dispensed from the drone, so that the local or global concentration of these agents can be adapted to specific needs. Typically, a faster air flow contributes to a better scattering of fragrances and/or pest repellents whereas a slower air flow creates a higher local concentration of pest repelling agents and/or fragrances.

According to an embodiment the at least one air treatment portion may comprise one among an air conditioning device, an air sanitizing device, an air humidifying device and an air filtering device.

According to an embodiment the at least one air treatment portion may comprise an ultraviolet radiation chamber.

According to an embodiment the at least one air treatment portion may comprise a replaceable air modifying element.

In particular, each air treatment portion may be removably arranged in the body of the drone along the air circulation pathway. In case of an air filtering system, a filter such as a fiber mat may be replaceable. For fragrance or pest repellent dispensing devices, cartridges comprising the fragrance or pest repellent may be removably arranged so as to only replace the cartridge during a refill or replacement operation.

According to an embodiment the processor may further be configured to receive input from a sensor, and the processor may be configured to determine a flight plan in the environment for the drone and an operation program for the at least one air ventilation mechanism based on the received input and the received instruction.

For example, information provided by a sensor can refer to detection of smoke due to the use of a kitchen appliance. The drone may then change its flight path to assist in removing the smoke from the kitchen of a household. Detection of a person smoking a cigarette, or activation of a barbecue may trigger a similar response for example. Detection of obstacles in an indoors environment or the presence of humans can also change the flight path of a drone, to avoid exposing humans to unpleasant pest repellants and to focus dispersion of fragrances in areas occupied by humans.

According to an embodiment the sensor may be at least one among:
- a temperature sensor;
- a humidity sensor;
- a detector of a chemical pollutant;
- a particulates detector;
- a barometer;
- a light intensity sensor;
- a virus detector,
- an aerosol collector.

According to an embodiment the drone may comprise a local sensor, the local sensor being at least one among:
- a motion sensor;
- a proximity sensor;
- a microphone;
- a camera.

The "local sensor" is a second sensors that can be found on the drone itself. A local sensor could also be located outside and provide the drone with information via a communication channel established with the drone. Typically, a camera could be used for facial recognition of users to adjust the operation of the drone to preset preferences of that user. A microphone can be used to receive vocal instructions from users or to listen to sounds produced to by pest to identify a pest type and/or identify its location. A proximity sensor such as a radar or sonar can be used to detect obstacles along the flight path of the drone. A motion sensor can be used to distinguish mobile objects from static objects and identify living creatures such as pets of humans and consequently adapt the flight path of the drone. The "local sensor" may be or may be coupled with a speaker to emit sounds or warnings in case of a hazardous situation (for example detection of a virus, an organic pollutant, a high concentration of pollutants or obstacles that require external intervention to allow the drone to fly into areas where it needs to perform an action).

According to an embodiment the drone may comprise a communications device capable of establishing a wireless communication with an external electronic device and capable of receiving one among sensor data and instructions to modify air properties.

The "external electronic device" can typically be a smartphone, a tablet, a smart watch, a computer or server.

The invention also pertains to a method for modifying air properties of an environment using a drone as described above. In particular such a drone comprises:
- a body;
- at least one air ventilation mechanism connected to the body;
- an air circulation pathway arranged in the body, the air circulation pathway being configured to suck in air and to expel air upon activation of the at least one air ventilation mechanism;
- steering means configured to maneuver the drone in air;
- at least one air treatment portion adapted for modifying air properties, the at least one air treatment portion being arranged in the body along the air circulation pathway;
- a processor, the processor being configured to control the steering means and to control the at least one air ventilation mechanism.

The method comprises:
- receiving an instruction to modify air properties in the environment
- determining, using the processor, a flight plan for the drone and an operation program for the at least one air ventilation mechanism based on the received instruction,
- controlling the steering means and controls the at least one air ventilation mechanism based on the instruction, so that the drone navigates through air in the environment and the at least one air ventilation mechanism creates a circulation of air through the at least one air treatment portion according to the determined flight plan and the determined operation program
- controlling flux of air channeled along the air circulation pathway and through the air treatment portion using several air flow intensity settings comprised in the at least one air ventilation mechanism.

According to an embodiment, the method may further comprise:
- receiving input from one among a sensor and an external communication device, and,
- modifying the flight plan for the drone and the operation program for the at least one air ventilation mechanism based on the received input.

The invention further pertains to a computer program product comprising instructions to cause a drone to execute a method for modifying air properties of an environment using a drone as described above, wherein the drone comprises:
- a body;
- at least one air ventilation mechanism connected to the body;
- an air circulation pathway arranged in the body, the air circulation pathway being configured to suck in air and to expel air upon activation of the at least one air ventilation mechanism;
- steering means configured to maneuver the drone in air;
- at least one air treatment portion adapted for modifying air properties, the at least one air treatment portion being arranged in the body along the air circulation pathway;
- a processor, the processor being configured to control the steering means and to control the at least one air ventilation mechanism;
the method comprising:
- receiving an instruction to modify air properties in the environment;
- determining, using the processor, a flight plan for the drone and an operation program for the at least one air ventilation mechanism based on the received instruction,
- outputting control signals for controlling the steering means and for controlling the at least one air ventilation mechanism based on the instruction, so that the drone navigates through air in the environment and the at least one air ventilation mechanism creates a circulation of air through the at least one air treatment portion according to the determined flight plan and the determined operation program
- controlling flux of air channeled along the air circulation pathway and through the air treatment portion using several air flow intensity settings comprised in the at least one air ventilation mechanism.

The above computer program product can also be viewed as a non-transitory computer readable storage medium having stored thereon a computer program comprising instructions for execution of the method described above.

### BRIEF DESRIPTION OF THE DRAWINGS

The present disclosure will hereinafter be described in conjunction with the following drawing figures, wherein like numerals denote like elements, and:
Fig. 1 is a schematic representation of a drone according to an embodiment;
Fig. 2 is a schematic representation of system comprising a drone according to an embodiment and external devices interacting with the drone.

### DETAILED DESCRIPTION

The invention provides a highly flexible and reactive device for modifying air properties using a drone. In particular, the invention can combine several actions performed on the air of an environment outdoors or indoors by comprising on a body of a drone at least one or more than one air treatment portions and by channeling air through the at least one air treatment portion along an air circulation pathway using at least one air ventilation mechanism.

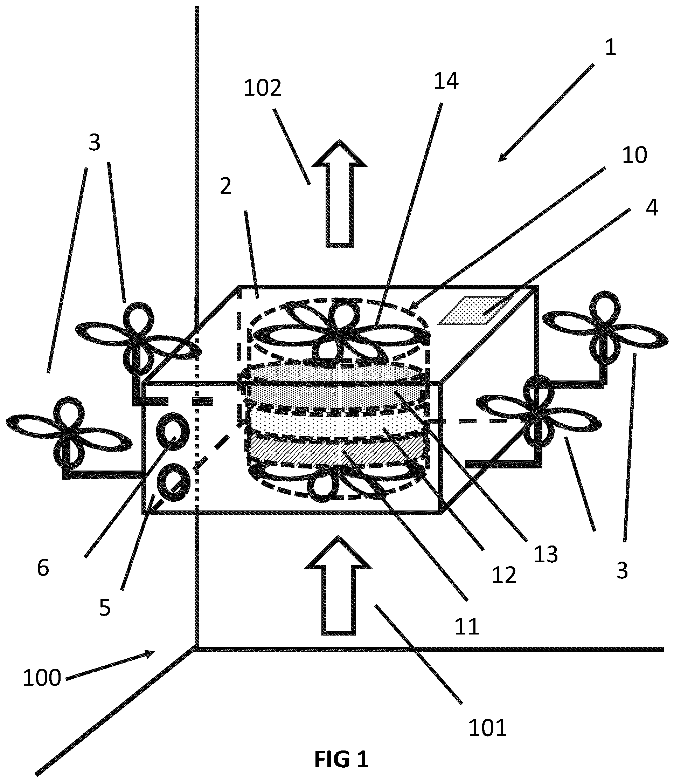

As can be seen on figure 1 a drone 1 according to an exemplary embodiment of the invention comprises a body 2, an air circulation pathway 10 that can for example be a hole within the body 2 of the drone 1 or a substantially tubular channel inside a closed volume attached to the body 2 of the drone 1.The drone 1 comprises steering means 3 typically in the form of propellers. The air circulation pathway 10 is obstructed at least partially by at least one air treatment portion 11, 12, 13, so that incoming air 101 is sucked into the air circulation pathway 10 and expelled air 102 exits the air circulation pathway 10 after passing through the at least one air treatment portion 11-13. To create this air circulation, at least one air ventilation mechanism 14 is arranged on or in the air circulation pathway 10. The at least one air ventilation mechanism 14 can typically be a fan.

The drone 1 can have any shape and size but is preferably a compact drone 1 having dimensions typically ranging between 10cm-30cm x 10cm-30cm x 10cm-30cm. It can be substantially rectangular in shape but can also be a spherical drone 1 with a diameter comprised typically between 15 and 40 cm.

The drone 1 can move around freely using the steering means 3. Control of these steering means as well as that of the at least one air ventilation mechanism 14 is enabled by the presence of a processor 4. The processor 4 is configured to receive instructions about a task to perform and based on these instructions determines a flight path for the drone 1 and a setting for the at least one air ventilation mechanism 14.

According to some embodiments, the processor 4 also controls the dosage of the dispensing of a composition that can be found in the at least one air treatment portions 11-13. For example the at least one air treatment portion 11 can be an air filtering system typically comprising a fiber mat, fibers or charcoal that can be replaced when too many particulates accumulate therein.

The at least one air treatment portion 12 can be an air sanitizing unit, for example a chamber comprising ultraviolet radiation sources (typically emitting light having wavelengths between 10 nm and 400nm, most preferably between 100 nm and 280 nm corresponding to so-called "ultraviolet C" radiation). The at least one air treatment portion 13 can for example be a fragrance dispensing device or a pest repellent dispensing device. The arrangement described above is advantageous as it enables performing three actions in a particular sequence during the flow of air from the environment 100 into the air circulation pathway 10: first the air is filtered to remove the large particulates such as dust present therein, then the air is sanitized by removing smells or chemicals or biological pollutants that can be harmful to users, lastly, the air properties are modified so as to repel pest and/or dispense a fragrance into the environment 100. More than three air treatment portions 11-13 or only one air treatment portion 11-13 can be found on the drone 1.

In other embodiments, the at least one air treatment portion 11-13 can be or can further comprise an air conditioning device, an air humidifying device and/or an air dehumidifying device.

It is to be noted that the environment 100 can be an indoor environment such as a house or apartment or an outdoor environment. In indoor environments, the drone can fly from room to room or determine a room in which a particular need to modify air properties is identified, such as removal of smoke in a kitchen or dining room, pest control in a living room (for example in the evening), bathroom or bedroom. The drone 1 may also comprise a patrolling mode in which it flies in different rooms in order to determine what to do next.

The instructions that define how the steering means 3 and the at least one air ventilation mechanism 14 are activated can be provided by a user or pre-programmed in the drone 1.

To assist the drone 1 in determining a current status of the environment in which it is located, the drone 1 may comprise at least one sensor 5 and at least one local sensor 6. The term "local sensor" refers to a sensor that is adapted to measure properties of the environment 100 such as for example the presence of objects on the flight path of the drone 1 (in which case the local sensor 6 is a proximity sensor), recognize objects or people (using a motion sensor or a camera for example), recognize noise generated by a pest or receive vocal commands (using a microphone for example), a global positioning system sensor (GPS) to determine the location of the drone and adapt the flight plan and activation of the at least one air ventilation mechanism 14 to specific conditions at a given geographical position.

The drone 1 may be further equipped with a speaker to emit sounds or warnings in case a hazardous situation is detected: the presence of pollutants above a safe level threshold, the presence of a virus considered as "dangerous" or putting users at risk of being infected, the presence of smoke. In case such a danger is detected, in addition to sanitizing or purifying air, the drone 1 may send a notification wirelessly to a user terminal such as an external communication device, such as a smartphone, table, smart watch, computer or server. The drone 1 may also or alternatively fly towards an area where a human is present to visually and or acoustically warn the user of the existing danger and recommend a course of action such as leave the environment 100 or call emergency services.

The at least one sensor 5 can for example comprise one among: a temperature sensor; a humidity sensor, a detector of one or more chemical pollutants such as organic volatile compounds (VOC), a particulates detector, a barometer, a light intensity sensor, a virus detector or a chamber for collecting aerosols which can be analyzed separately in a laboratory, or directly at the drone 1 using chemical reactants or spectroscopic devices mounted on or in the drone 1. By detecting the presence of pollutants or allergens, the drone 1 can adapt its air properties modification strategy to eliminate the identified nuisance or threat even if it is already accomplishing a preprogrammed task.

In some exemplary embodiments. the drone 1 can for example fly outdoors and assist in removing smoke or smells in outdoor gatherings such as barbecues. The drone may also navigate in the outdoor environment in order to identify the presence of smokers and filter air if the smoke accumulates. Otherwise, the drone may remove smells from barbecues.

In indoor environments, the drone 1 may track the presence of smells and assist in the removal of smells in kitchens or the dispensing of fragrance and purification of air in living rooms and bedrooms. It can also identify the presence of people and dispense a fragrance based on the number or profile of the person that is identified. Preset preferences for fragrances or times of day or weather conditions for their dispensing that are programmed in the drone 1 by a user can be read to adapt the flight path and dispensing strategy (by controlling the steering means 3 and the at least one air ventilation mechanism 14).

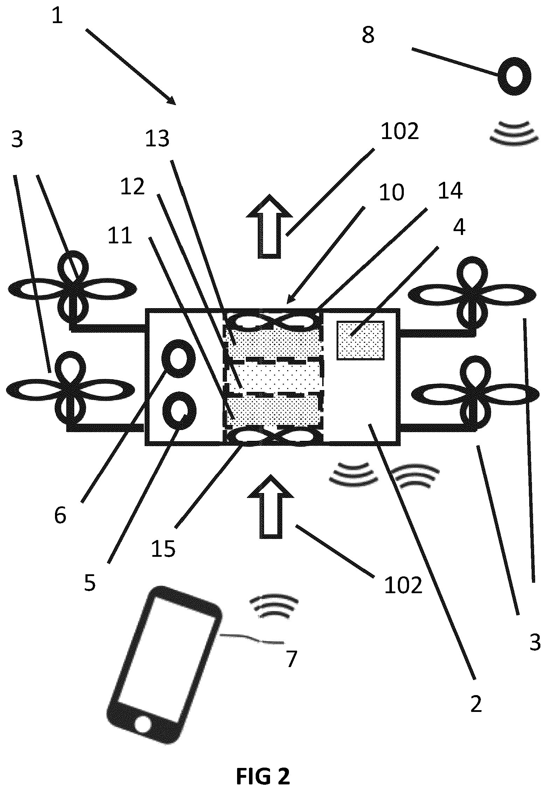

Figure 2 shows the drone of figure 1 in a configuration in which the drone 1 exchanges information with external devices such as an external communication device 7, for example a smartphone, tablet or smart watch. Similarly, the drone 1 can receive information from an external sensor 8 for example a weather station, a thermometer, a barometer or any sensor capable of sending information the processor of the drone 1 would then use to adapt its flight plan and setting of the at least one air ventilation mechanism 14, 15.

The at least one air ventilation mechanism 14, 15 can comprise, as represented on figure 2, two or more fans. Direction of air flow can be modified by the processor 4 by changing the direction in which the at least one air ventilation mechanism 14, 15 rotates. A speed of rotation of the at least one air ventilation mechanism 14, 15 can also be adjusted by the processor 4. Higher rotation speeds create a faster air flow through the air circulation pathway 10 which can contribute to a better scattering of any fragrance or pest repellent dispensed into the environment 100. Slower rotation speeds create a slower air flow through the air circulation pathway 10 which can be used for example to increase a local concentration of fragrance or pest repellent.

The activation and setting of the at least one air ventilation mechanism 14, 15, the flight path of the drone 1 can be set manually by a user or can be set automatically after determination of the most suitable settings by the processor 4. Determination can occur externally and only communicated after completion to the processor 4 for execution of the instructions.

Dosage of the amount of fragrance and/or pest repellent can also be determined by the processor 4 and controlled by the processor 4 or controlled by the processor 4 and determined at an external computing unit. A user can also order a specific dosage or select a dosage intensity or strategy for the dispensing of the fragrance and/or pest repellent. For example one dispensing strategy may be to dispense a minimal amount of fragrance and/or pest repellent during the night and increase the amount dispensed upon detecting the presence of pest or undesired smells, the amount dispensed being increased as the drone 1 flies towards the area that requires the fragrance and/or pest repellent to be dispensed. It is to be noted that the dispensing can occur in a continuous or discontinuous fashion.

Among pest repellents compositions such as methylnonylketone, icaridine, DEET, SS220, NMK, methyl anthranilate, terpenes such as citronellol or mixtures thereof can be used. It is also possible to mix pest repellents with fragrances.

According to an autonomous mode of the drone 1, a monitoring of the battery status of the drone 1 can be implemented on a continuous or discontinuous basis and an order to fly back to a docking and charging station can be programmed in the drone when the battery power falls below a predetermined threshold.

The invention also pertains to a method for modifying air properties using a drone as described above. Such a method consists in:
- receiving an instruction to modify air properties in the environment,
- determining, using the processor 4, a flight plan for the drone and an operation program for the at least one air ventilation mechanism 14 based on the received instruction,
- controlling the steering means 3 and control the at least one air ventilation mechanism 14 based on the instruction, so that the drone navigates through air in the environment 100 and the at least one air ventilation mechanism 14 creates a circulation of air through the at least one air treatment portion 11-13 according to the determined flight plan and the determined operation program.

The method can be viewed as a set of instructions to be performed by the processor and which can be stored on a non-transitory computer-readable storage medium in the form of a computer program.

The steps of the examples and embodiments described above can be implemented by a processor such as a computer. A computer program product comprising steps of the above-described method can be used to implement the method on a computer.

It is possible to store a computer program comprising instructions to implement the method of the invention on different non-transitory computer readable storage mediums. These could for example comprise a processor or chip, FPGA (field programable gate array), an electronic circuit comprising several processors or chips, a hard drive, a flash or SD card, a USB stick, a CD-ROM or DVD-ROM or Blue-Ray disc, or a diskette.

While at least one exemplary embodiment has been presented in the foregoing detailed description, it should be appreciated that a vast number of variations exist. It should also be appreciated that the exemplary embodiment or exemplary embodiments are only examples, and are not intended to limit the scope, applicability, or configuration of the various embodiments in any way. Rather, the foregoing detailed description will provide those skilled in the art with a convenient road map for implementing an exemplary embodiment as contemplated herein. It being understood that various changes may be made in the function and arrangement of elements described in an exemplary embodiment without departing from the scope of the various embodiments as set forth in the appended claims.

## Claims

1. A drone (1) adapted for modifying air properties of an environment (100), the drone comprising:
- a body (2);
- at least one air ventilation mechanism (14, 15) connected to the body;
- an air circulation pathway (10) arranged in the body, the air circulation pathway being configured to suck in air and to expel air upon activation of the at least one air ventilation mechanism;
- steering means (3) configured to maneuver the drone in air;
- at least one air treatment portion (11, 12, 13) adapted for modifying air properties, the at least one air treatment portion being arranged in the body along the air circulation pathway;
- a processor (4), the processor being configured to control the steering means and to control the at least one air ventilation mechanism;
wherein the processor is adapted to receive an instruction to modify air properties and
wherein the processor controls the steering means and controls the at least one air ventilation mechanism based on the instruction, so that the drone navigates through air in the environment and the at least one air ventilation mechanism creates a circulation of air through the at least one air treatment portion
wherein the at least one air ventilation mechanism (14, 15) comprises several air flow intensity settings for controlling flux of air channeled along the air circulation pathway (10) and through the air treatment portion (11, 12, 13).

2. The drone according to any one of the preceding claims, wherein the at least one air treatment portion (13) comprises a fragrance dispensing device.

3. The drone according to any one of the preceding claims, wherein the at least one air treatment portion (13) comprises a pest repellent.

4. The drone according to any one of the preceding claims, wherein the processor (4) further controls dispensing of an air modifying composition in the at least one air treatment portion (11, 12, 13).

5. The drone according to any one of the preceding claims, wherein the at least one air treatment portion (11, 12, 13) comprises one among an air conditioning device, an air sanitizing device, an air humidifying device and an air filtering device.

6. The drone according to any one of the preceding claims, wherein the at least one air treatment portion (12) comprises an ultraviolet radiation chamber.

7. The drone according to any one of the preceding claims, wherein the at least one air treatment portion (11) comprises a replaceable air modifying element.

8. The drone according to any one of the preceding claims, wherein the processor (4) is further configured to receive input from a first sensor (5), and wherein the processor is configured to determine a flight plan in the environment for the drone (1) and an operation program for the at least one air ventilation mechanism (14, 15) based on the received input and the received instruction.

9. The drone according to any one of the preceding claims, wherein the first sensor (5) is at least one among:
- a temperature sensor;
- a humidity sensor;
- a detector of a chemical pollutant;
- a particulates detector;
- a barometer;
- a light intensity sensor;
- a virus detector,
- an aerosol collector.

10. The drone according to any one of the preceding claims, wherein the drone (1) comprises a local sensor as second sensor (6), the second sensor being at least one among:
- a motion sensor;
- a proximity sensor;
- a microphone;
- a camera.

11. The drone according to any one of the preceding claims, wherein the drone (1) comprises a communications device capable of establishing a wireless communication with an external electronic device (7) and capable of receiving one among sensor data and instructions to modify air properties.

12. A method for modifying air properties of an environment using a drone (1), wherein the drone comprises:
- a body (2);
- at least one air ventilation mechanism (14, 15) connected to the body;
- an air circulation pathway (10) arranged in the body, the air circulation pathway being configured to suck in air and to expel air upon activation of the at least one air ventilation mechanism;
- steering means (3) configured to maneuver the drone in air;
- at least one air treatment portion (11, 12, 13) adapted for modifying air properties, the at least one air treatment portion being arranged in the body along the air circulation pathway;
- a processor (4), the processor being configured to control the steering means and to control the at least one air ventilation mechanism;
the method comprising:
- receiving an instruction to modify air properties in the environment
- determining, using the processor, a flight plan for the drone and an operation program for the at least one air ventilation mechanism based on the received instruction,
- controlling the steering means and controls the at least one air ventilation mechanism based on the instruction, so that the drone navigates through air in the environment and the at least one air ventilation mechanism creates a circulation of air through the at least one air treatment portion according to the determined flight plan and the determined operation program
- controlling flux of air channeled along the air circulation pathway (10) and through the air treatment portion (11, 12, 13) using several air flow intensity settings comprised in the at least one air ventilation mechanism (14, 15).

13. The method according to claim 12, further comprising:
- receiving input from one among a sensor (5, 8) and an external communication device (7), and,
- modifying the flight plan for the drone (1) and the operation program for the at least one air ventilation mechanism (11, 12, 13) based on the received input.

14. Computer program product comprising instructions to cause a drone to execute the steps of a method for modifying air properties of an environment, wherein the drone comprises:
- a body (1);
- at least one air ventilation mechanism (14, 15) connected to the body;
- an air circulation pathway (10) arranged in the body, the air circulation pathway being configured to suck in air and to expel air upon activation of the at least one air ventilation mechanism;
- steering means (3) configured to maneuver the drone in air;
- at least one air treatment portion (11, 12, 13) adapted for modifying air properties, the at least one air treatment portion being arranged in the body along the air circulation pathway;
- a processor (4), the processor being configured to control the steering means and to control the at least one air ventilation mechanism;
the method comprising:
- receiving an instruction to modify air properties in the environment;
- determining, using the processor, a flight plan for the drone and an operation program for the at least one air ventilation mechanism based on the received instruction,
- outputting control signals for controlling the steering means and for controlling the at least one air ventilation mechanism based on the instruction, so that the drone navigates through air in the environment and the at least one air ventilation mechanism creates a circulation of air through the at least one air treatment portion according to the determined flight plan and the determined operation program
- controlling flux of air channeled along the air circulation pathway (10) and through the air treatment portion (11, 12, 13) using several air flow intensity settings comprised in the at least one air ventilation mechanism (14, 15).

## Patentansprüche

1. Eine Drohne (1), die dazu ausgelegt ist, die Lufteigenschaften einer Umgebung (100) zu verändern, wobei die Drohne umfasst:
- einen Rumpf (2);
- mindestens einen mit dem Gehäuse verbundenen Belüftungsmechanismus (14, 15);
- einen im Rumpf angeordneten Luftzirkulationskanal (10), wobei der Luftzirkulationskanal so ausgelegt ist, dass er bei Aktivierung des mindestens einen Belüftungsmechanismus Luft ansaugt und Luft ausstößt;
- Steuermittel (3), die so ausgelegt sind, dass sie die Drohne in der Luft manövrieren;
- mindestens einen Luftaufbereitungsabschnitt (11, 12, 13), der zur Veränderung von Lufteigenschaften ausgelegt ist, wobei der mindestens eine Luftaufbereitungsabschnitt im Gehäuse entlang des Luftzirkulationswegs angeordnet ist;
- einen Prozessor (4), wobei der Prozessor so ausgelegt ist, dass er die Steuermittel und den mindestens einen Belüftungsmechanismus steuert;
wobei der Prozessor dazu ausgelegt ist, einen Befehl zur Veränderung der Lufteigenschaften zu empfangen, und wobei der Prozessor die Steuermittel und den mindestens einen
Belüftungsmechanismus auf der Grundlage des Befehls so steuert, dass die Drohne durch die Luft in der Umgebung navigiert und der mindestens eine Belüftungsmechanismus eine Luftzirkulation durch den mindestens einen Luftaufbereitungsabschnitt erzeugt,
wobei der mindestens eine Luftbelüftungsmechanismus mehrere Luftstromintensitätsstufen umfasst, um den Luftstrom zu steuern, der entlang des Luftzirkulationswegs und durch den Luftaufbereitungsabschnitt geleitet wird.

2. Die Drohne gemäß einem der vorstehenden Ansprüche, wobei die mindestens eine Luftbehandlungsabschnitt eine Duftstoffabgabevorrichtung umfasst.

3. Die Drohne nach einem der vorstehenden Ansprüche, wobei der mindestens eine Luftbehandlungsabschnitt ein Schädlingsabwehrmittel umfasst.

4. Die Drohne nach einem der vorstehenden Ansprüche, wobei der Prozessor ferner die Abgabe einer luftverändernden Zusammensetzung in dem mindestens einen Luftaufbereitungsabschnitt steuert.

5. Drohne nach einem der vorstehenden Ansprüche, wobei der mindestens eine Luftaufbereitungsabschnitt eine der folgenden Einrichtungen umfasst: eine Klimaanlage, eine Luftdesinfektionsvorrichtung, eine Luftbefeuchtungsvorrichtung oder eine Luftfiltervorrichtung.

6. Drohne nach einem der vorstehenden Ansprüche, wobei der mindestens eine Luftaufbereitungsabschnitt eine UV-Strahlungskammer umfasst.

7. Die Drohne nach einem der vorstehenden Ansprüche, wobei der mindestens eine Luftbehandlungsabschnitt ein austauschbares Luftmodifikationselement umfasst.

8. Die Drohne nach einem der vorstehenden Ansprüche, wobei der Prozessor ferner so konfiguriert ist, dass er Eingaben von einem ersten Sensor (5) empfängt, und wobei der Prozessor so konfiguriert ist, dass er einen Flugplan in der Umgebung für die Drohne und ein Betriebsprogramm für den mindestens einen Belüftungsmechanismus auf der Grundlage auf der empfangenen Eingabe und der empfangenen Anweisung.

9. Die Drohne gemäß einem der vorstehenden Ansprüche, wobei der erste Sensor mindestens einer der folgenden ist:
- ein Temperatursensor;
- ein Feuchtigkeitssensor;
- einen Detektor für chemische Schadstoffe;
- ein Partikeldetektor;
- ein Barometer;
- einen Lichtintensitätssensor;
- einen Virusdetektor,
- einen Aerosolsammler.

10. Die Drohne gemäß einem der vorstehenden Ansprüche, wobei die Drohne einen zweiten Sensor (6) umfasst, wobei der zweite Sensor mindestens einer der folgenden ist:
- einen Bewegungssensor;
- einen Näherungssensor;
- ein Mikrofon;
- eine Kamera.

11. Drohne nach einem der vorstehenden Ansprüche, wobei die Drohne eine Kommunikationseinrichtung umfasst, die in der Lage ist, eine drahtlose Kommunikation mit einem externen elektronischen Gerät herstellen und entweder Sensordaten oder Anweisungen zur Veränderung der Lufteigenschaften empfangen kann.

12. Verfahren zur Veränderung der Lufteigenschaften einer Umgebung unter Verwendung einer Drohne (1),
wobei die Drohne umfasst:
- einen Rumpf (2);
- mindestens einen mit dem Gehäuse verbundenen Luftumwälzmechanismus (14, 15);
- einen im Gehäuse angeordneten Luftzirkulationskanal (10), wobei der Luftzirkulationskanal so ausgelegt ist, dass er bei Aktivierung des mindestens einen Belüftungsmechanismus;
- Steuermittel (3), die so ausgelegt sind, dass sie die Drohne in der Luft manövrieren;
- mindestens einen Luftaufbereitungsabschnitt (11, 12, 13), der zur Veränderung der Lufteigenschaften ausgelegt ist, wobei der mindestens eine Luftaufbereitungsabschnitt im Gehäuse entlang des Luftzirkulationswegs angeordnet ist;
- einen Prozessor (4), wobei der Prozessor so ausgelegt ist, dass er die Steuerungsvorrichtung und den mindestens einen Belüftungsmechanismus steuert;
wobei das Verfahren umfasst:
- Empfangen eines Befehls zur Veränderung der Lufteigenschaften in der Umgebung
- Ermitteln eines Flugplans für die Drohne und eines Betriebs für den mindestens einen Belüftungsmechanismus auf der Grundlage des empfangenen Befehls,
- , der die Lenkeinrichtung steuert und den mindestens einen Belüftungsmechanismus auf der Grundlage der Anweisung so steuert, dass die Drohne durch die Luft in der Umgebung navigiert und der mindestens eine Belüftungsmechanismus eine Zirkulation
von Luft durch den mindestens einen Luftaufbereitungsabschnitt gemäß dem festgelegten Flugplan und dem festgelegten Betriebsprogramm
- Steuerung des Luftstroms, der entlang des Luftzirkulationswegs und durch den Luftaufbereitungsabschnitt geleitet wird, unter Verwendung mehrerer Luftstromintensitätseinstellungen, die in dem mindestens einen Belüftungsmechanismus enthalten sind.

13. Verfahren nach Anspruch 12, das ferner umfasst:
- Empfangen von Eingaben von entweder einem Sensor (5, 8) oder einem externen Kommunikationsgerät (7) und
- Ändern des Flugplans für die Drohne und des Betriebsprogramms für den mindestens Lüftungsmechanismus auf der Grundlage der empfangenen Eingabe.

14. Computerprogrammprodukt, das Anweisungen zur Ausführung eines Verfahrens zum Ändern der Lufteigenschaften einer Umgebung unter Verwendung einer Drohne umfasst, wobei die Drohne Folgendes umfasst:
- einen Körper (1);
- mindestens einen mit dem Gehäuse verbundenen Belüftungsmechanismus (14, 15);
- einen im Gehäuse angeordneten Luftzirkulationskanal (10), wobei der Luftzirkulationskanal so ausgelegt ist, dass er bei Aktivierung des mindestens einen Belüftungsmechanismus Luft ansaugt und Luft ausstößt;
- Steuermittel (3), die so ausgelegt sind, dass sie die Drohne in der Luft manövrieren;
- mindestens einen Luftaufbereitungsabschnitt (11, 12, 13), der zur Veränderung von Lufteigenschaften ausgelegt ist, wobei der mindestens eine Luftaufbereitungsabschnitt im Gehäuse entlang des Luftzirkulationswegs angeordnet ist;
- einen Prozessor (4), wobei der Prozessor so ausgelegt ist, dass er die Steuermittel und den mindestens einen Belüftungsmechanismus steuert;
wobei das Verfahren umfasst:
- Empfangen einer Anweisung zur Veränderung der Lufteigenschaften in der Umgebung;
- Ermitteln eines Flugplans für die Drohne und eines Betriebsprogramms für den mindestens einen Luftbelüftungsmechanismus unter Verwendung des Prozessors auf der Grundlage des empfangenen Befehls,
- Ausgeben von Steuersignalen zur Steuerung der Lenkeinrichtung und zur Steuerung des mindestens einen Belüftungsmechanismus auf der Grundlage der Anweisung, sodass die Drohne durch die Luft in der Umgebung navigiert und der mindestens eine Belüftungsmechanismus eine Luftzirkulation durch den mindestens einen Luftaufbereitungsabschnitt
gemäß dem festgelegten Flugplan und dem festgelegten Betriebsablauf Programm
- , der den entlang des Luftzirkulationswegs und durch den Luftaufbereitungsabschnitt geleiteten Luftstrom unter Verwendung mehrerer Luftstromintensitätseinstellungen steuert, die in dem mindestens einen Belüftungsmechanismus enthalten sind.

## Revendications

1. Un drone (1) adapté pour modifier les propriétés de l'air d'un environnement (100), le drone comprenant :
- un corps (2) ;
- au moins un mécanisme de ventilation (14, 15) relié au corps ;
- un circuit de circulation d'air (10) disposé dans le corps, ledit circuit de circulation d'air étant configuré pour aspirer de l'air et pour expulser de l'air lors de l'activation dudit au moins un mécanisme de ventilation ;
- des moyens de pilotage (3) configurés pour manœuvrer le drone dans les airs
- au moins une partie de traitement de l'air (11, 12, 13) adaptée pour modifier les propriétés de l'air, ladite au moins une partie de traitement de l'air étant disposée dans le corps le long du circuit de circulation d'air ;
- un processeur (4), le processeur étant configuré pour commander les moyens de pilotage et pour commander le au moins un mécanisme de ventilation d'air
dans lequel le processeur est adapté pour recevoir une instruction visant à modifier les propriétés de l'air et dans lequel le processeur commande les moyens de pilotage et commande le au moins un
en fonction de cette instruction, de sorte que le drone se déplace dans l'air de l'environnement et que le au moins un mécanisme de ventilation crée une circulation d'air à travers la au moins une partie de traitement de l'air
dans lequel ledit au moins un mécanisme de ventilation d'air comprend plusieurs réglages d'intensité de débit d'air pour commander le flux d'air acheminé le long du trajet de circulation d'air et à travers la partie de traitement de l'air.

2. Drone selon l'une quelconque des revendications précédentes, dans lequel ledit au moins un composante de traitement de l'air comprend un dispositif de diffusion de parfum.

3. Le drone selon l'une quelconque des revendications précédentes, dans lequel la au moins une partie de traitement de l'air comprend un répulsif contre les nuisibles.

4. Le drone selon l'une quelconque des revendications précédentes, dans lequel le processeur commande en outre la diffusion d'une composition modifiant l'air dans la au moins une partie de traitement de l'air.

5. Le drone selon l'une quelconque des revendications précédentes, dans lequel la au moins une partie de traitement de l'air comprend l'un parmi un dispositif de climatisation, un dispositif d'assainissement de l'air, un dispositif d'humidification de l'air et un dispositif de filtration de l'air.

6. Le drone selon l'une quelconque des revendications précédentes, dans lequel la au moins une partie de traitement de l'air comprend une chambre de rayonnement ultraviolet.

7. Le drone selon l'une quelconque des revendications précédentes, dans lequel la au moins une partie de traitement de l'air comprend un élément de modification de l'air remplaçable.

8. Le drone selon l'une quelconque des revendications précédentes, dans lequel le processeur est en outre configuré pour recevoir des données provenant d'un premier capteur (5), et dans lequel le processeur est configuré pour déterminer un plan de vol dans l'environnement pour le drone et un programme de fonctionnement pour le au moins un mécanisme de ventilation de l'air en fonction
sur les données reçues et l'instruction reçue.

9. Drone selon l'une quelconque des revendications précédentes, dans lequel le premier capteur est au moins l'un des éléments suivants :
- un capteur de température ;
- un capteur d'humidité ;
- un détecteur de polluants chimiques ;
- un détecteur de particules ;
- un baromètre ;
- un capteur d'intensité lumineuse ;
- un détecteur de virus,
- un collecteur d'aérosols.

10. Le drone selon l'une quelconque des revendications précédentes, dans lequel le drone comprend un deuxième capteur (6), le deuxième capteur étant au moins l'un parmi :
- un capteur de mouvement ;
- un capteur de proximité ;
- un microphone ;
- une caméra.

11. Drone selon l'une quelconque des revendications précédentes, **caractérisé en ce qu'**il comprend un dispositif de communication capable d'établir une avec un dispositif électronique externe et capable de recevoir soit des données de capteurs, soit des instructions visant à modifier les propriétés de l'air.

12. Procédé permettant de modifier les propriétés de l'air d'un environnement à l'aide d'un drone (1),
dans lequel le drone comprend :
- un corps (2) ;
- au moins un mécanisme de ventilation d'air (14, 15) relié au corps ;
- un circuit de circulation d'air (10) disposé dans le corps, ledit circuit de circulation d'air étant configuré pour aspirer de l'air et pour expulser de l'air lors de l'activation dudit au moins
un mécanisme de ventilation d'air ;
- des moyens de pilotage (3) configurés pour manœuvrer le drone dans les airs
;
- au moins une partie de traitement de l'air (11, 12, 13) adaptée pour modifier les propriétés de l'air, ladite au moins une partie de traitement de l'air étant disposée dans le corps le long du trajet de circulation de l'air ;
- un processeur (4), ledit processeur étant configuré pour commander les moyens de pilotage et pour commander le ou les mécanismes de ventilation d'air
le procédé comprenant :
- la réception d'une instruction visant à modifier les propriétés de l'air dans l'environnement
- déterminer, à l'aide du processeur, un plan de vol pour le drone et un programme de fonctionnement pour le au moins un mécanisme de ventilation d'air sur la base de l'instruction reçue,
- commande les moyens de pilotage et contrôle le ou les mécanismes de ventilation d'air en fonction de l'instruction, de sorte que le drone se déplace dans l'air de l'environnement et que le ou les mécanismes de ventilation d'air créent une circulation
d'air à travers la au moins une partie de traitement de l'air conformément au plan de vol déterminé et au programme de fonctionnement déterminé
- contrôler le flux d'air acheminé le long du trajet de circulation d'air et à travers la partie de traitement de l'air à l'aide de plusieurs réglages d'intensité du débit d'air compris dans le au moins un mécanisme de ventilation.

13. Procédé selon la revendication 12, comprenant en outre :
- la réception d'une entrée provenant soit d'un capteur (5, 8), soit d'un dispositif de communication externe (7), et,
- la modification du plan de vol du drone et du programme de fonctionnement de la au moins
mécanisme de ventilation, sur la base des données reçues.

14. Produit logiciel comprenant des instructions pour exécuter un procédé de modification des propriétés de l'air d'un environnement à l'aide d'un drone, dans lequel le drone comprend :
- un corps (1) ;
- au moins un mécanisme de ventilation (14, 15) relié au corps ;
- un circuit de circulation d'air (10) disposé dans le corps, ledit circuit de circulation d'air étant configuré pour aspirer de l'air et pour expulser de l'air lors de l'activation dudit au moins un mécanisme de ventilation ;
- des moyens de pilotage (3) configurés pour manœuvrer le drone dans les airs ;
- au moins une partie de traitement de l'air (11, 12, 13) adaptée pour modifier les propriétés de l'air, ladite au moins une partie de traitement de l'air étant disposée dans le corps le long du circuit de circulation d'air ;
- un processeur (4), le processeur étant configuré pour commander les moyens de pilotage et pour commander le au moins un mécanisme de ventilation d'air ;
le procédé comprenant :
- la réception d'une instruction visant à modifier les propriétés de l'air dans l'environnement ;
- déterminer, à l'aide du processeur, un plan de vol pour le drone et un programme de fonctionnement pour le au moins un mécanisme de ventilation de l'air sur la base de l'instruction reçue,
- en émettant des signaux de commande destinés à commander les moyens de direction et à commander le ou les mécanismes de ventilation, sur la base de l'instruction, de sorte que le drone se déplace dans l'air au sein de l'environnement et que le ou les mécanismes de ventilation créent une circulation d'air à travers la ou les parties de traitement de l'air
conformément au plan de vol déterminé et au programme de fonctionnement déterminé
- contrôlant le flux d'air acheminé le long du parcours de circulation d'air et à travers la partie de traitement de l'air à l'aide de plusieurs réglages d'intensité du débit d'air compris dans le au moins un mécanisme de ventilation.
